# EUROPEAN PATENT APPLICATION

(11) **EP 4 674 863 A1**
(43) Date of publication of application: **07.01.2026**
(21) Application number: 23933890.8
(22) Date of filing: 18.12.2023
(51) Int. Cl.: C07K 14/155, C12N 15/867, C12N 15/55, C12N 15/113, A61K 48/00, A61P 25/14

(54) **LENTIVIRUS-LIKE PARTICLE FOR TREATING HUNTINGTON'S DISEASE**

(30) Priority: 18.04.2023 CN 202310418451
(71) Applicant: Shanghai Bdgene Co., Ltd, Shanghai 200245 (CN)
(72) Inventor: LING, Sikai, Shanghai 200245 (CN); ZHANG, Lin, Shanghai 200245 (CN)
(74) Representative: Bayramoglu et al.
(86) International application number: PCT/CN2023/139427
(87) International publication number: WO 2024/217027

(57) **Abstract**

The present disclosure provides a lentivirus-like particle for treating Huntington's disease (HD). The lentivirus-like particle VLP-HD is produced by encapsulating an RNP complex including a Cas9 protein and an *HTT* gene-targeted gRNA with a virus-like particle (VLP) vector. After infecting cells, VLP-HD releases Cas9:gRNA RNP, which can efficiently achieve the targeted knockout of the mutant *HTT* gene. The present disclosure can prevent the production of the mutant HTT protein with PolyQ at the source, thereby achieving the etiological therapy for HD. VLP-HD can be either single-target VLP that delivers a single CRISPR/Cas9 system to knock out the mutant gene through a frameshift mutation of the m*HTT* gene, or dual-target VLP that delivers two CRISPR/Cas9 systems to delete disease-causing CAG trinucleotide repeats in the *mHTT* gene, thereby completely avoiding the production of PolyQ.

## Description

### TECHNICAL FIELD

The present disclosure belongs to the field of gene therapy, and relates to a lentivirus-like particle for treating Huntington's disease (HD). In particular, the present disclosure relates to a modified lentivirus-like particle (VLP) for treating HD.

### BACKGROUND TECHNOLOGY

Huntington's disease (HD) is an inherited neurological disorder characterized by psychiatric disturbances, cognitive impairment, and involuntary choreiform movements. HD is an autosomal dominant genetic disorder caused by expanded CAG trinucleotide repeats within exon 1 of the *HTT* gene on autosome 4. The HTT protein expressed by the *HTT* gene is widely expressed in human neurons and possesses diverse functions. In healthy individuals, the *HTT* gene includes 17 to 20 CAG repeats on average. When there are 40 or more CAG repeats, polyglutamine (PolyQ) is produced, resulting in the aggregation of the huntingtin protein. Protein aggregates cause the dysregulation of gene transcription when entering the nucleus. Protein aggregates disrupt the proteostasis in the cytoplasm, including synaptic dysfunction, mitochondrial toxicity, and reduced axonal transport efficiency. As a result, the protein aggregation leads to the occurrence of HD. HD patients typically develop cognitive and motor disorders in middle age, which are aggravated with advancing age.

The global prevalence of HD is approximately 2.7 per 100,000 individuals. In western countries, the prevalence of HD is 10.6 to 13.7 per 100,000 individuals. Currently, the clinical management for HD involves only symptomatic therapy, but involves no etiological therapy. With the technical development in gene therapy, the etiological therapy has become possible for genetic disorders caused by gene mutations. Recently, the therapy AMT-130 in which microRNA is delivered by an adeno-associated virus (AAV) vector (rAAV5-miHTT) to knock down the mutant HTT (m*HTT*) gene has been proposed for the treatment of HD. The phase II clinical trial (NCT05243017) is currently underway to investigate the safety and efficacy of AMT-130 in adult patients with early HD in Europe. The therapeutic strategy for this therapy is as follows: The long-term expression of m*HTT* gene-targeted microRNA in neurons is achieved through an AAV vector to suppress the translation of m*HTT* mRNA, thereby knocking down the mutant protein. However, this method requires the long-term colonization of the exogenous gene in cells. The stability of the exogenous gene is unknown, and the exogenous gene may be integrated into a genome of a host cell.

### CONTENT OF THE INVENTION

An objective of the present disclosure is to address the safety and efficacy issues of the treatment of HD in the prior art and provide a lentivirus-like particle for treating HD. Specifically, this treatment method is a gene-editing therapy, and is achieved by delivering CRISPR RNP with VLP to allow the targeted knockout of the *HTT* gene.

Specifically, the objective of the present disclosure is achieved by the following technical solutions:

### <First Aspect>

The present disclosure relates to a lentivirus-like particle including: a VLP vector, and an RNP complex including a Cas9 protein and an *HTT* gene-targeted gRNA. The lentivirus-like particle VLP is produced by encapsulating the RNP complex including the Cas9 protein and the targeting gRNA with a modified lentiviral envelope. The modified lentiviral envelope is produced by fusing an RNA binding protein to a lentiviral GagPol polyprotein and then packaging.

As an embodiment of the present disclosure, gene sequences expressing components in the VLP vector are located on (1) a plasmid expressing an envelope protein, (2) a plasmid expressing a lentiviral GagPol polyprotein including an RNA binding protein, (3) a plasmid expressing a lentiviral GagPol polyprotein, (4) a helper plasmid pRSV-Rev, (5) a plasmid expressing the Cas9 protein required to produce the RNP complex, and (6) a plasmid expressing gRNA including an RNA stem-loop structure recognizable by the RNA binding protein, respectively.

As an embodiment of the present disclosure, the plasmid expressing the Cas9 protein required to produce the RNP complex and the plasmid expressing the gRNA including the RNA stem-loop structure recognizable by the RNA binding protein can be a same plasmid. That is, the gene sequences expressing the components in the VLP vector are located on (1) a plasmid expressing an envelope protein, (2) a plasmid expressing a lentiviral GagPol polyprotein including an RNA binding protein, (3) a plasmid expressing a lentiviral GagPol polyprotein, (4) a helper plasmid pRSV-Rev, and (5) a plasmid expressing the Cas9 protein required to produce the RNP complex and gRNA including an RNA stem-loop structure recognizable by the RNA binding protein, respectively.

As an embodiment of the present disclosure, the envelope protein among the components in the VLP vector includes VSV-G, a CD4-recognizing protein, a CD8-recognizing protein, RD114, a baboon endogenous retrovirus envelope protein, or a modified envelope protein with cell-specific infectivity.

As an embodiment of the present disclosure, an amino acid sequence of the VSV-G is shown in SEQ ID NO. 7.

As an embodiment of the present disclosure, the modified envelope protein with cell-specific infectivity is an NVR-G protein with neuron-specific infectivity, and an amino acid sequence of the NVR-G protein is shown in SEQ ID NO. 8.

As an embodiment of the present disclosure, a gene sequence for the NVR-G protein is shown in SEQ ID NO. 29.

As an embodiment of the present disclosure, the lentiviral GagPol polyprotein including the RNA binding protein has an amino acid sequence shown in SEQ ID NO. 9.

As an embodiment of the present disclosure, a scaffold sequence of the gRNA including the RNA stem-loop structure recognizable by the RNA binding protein is shown in SEQ ID NO. 10.

As an embodiment of the present disclosure, a targeting site of a guide sequence carried by the HTT gene-targeted gRNA can be any site on the *HTT* gene. VLP carrying one Cas9:gRNA complex targets the mutant *HTT* (mHTT) gene to produce Indel (insertion/deletion) and cause a frameshift mutation, thereby knocking out the mutant *HTT* (m*HTT*) gene. VLP carrying two Cas9:gRNA complexes targets two ends of the *HTT* gene at a CAG repeat to break the CAG repeat, thereby preventing the production of protein aggregates.

As an embodiment of the present disclosure, the *HTT* gene-targeted gRNA is:
gRNA1: 5'-GGCCTTCATCAGCTTTTCCA-3',
gRNA4: 5'-GAAGGACTTGAGGGACTCGA-3',
gRNA7: 5'-GACCCTGGAAAAGCTGATGA-3',
gRNA8: 5'-GGAGACCGCCATGGCGACCC-3',
gRNA9: 5'-CAGCTTTTCCAGGGTCGCCA-3',
gRNA10: 5'-CTTTTCCAGGGTCGCCATGG-3', or
gRNA11: 5'-AGGCCTTCATCAGCTTTTCC-3'.

As an embodiment of the present disclosure, the *HTT* gene-targeted gRNA includes at least two selected from the following sequences:
gRNA1: 5'-GGCCTTCATCAGCTTTTCCA-3',
gRNA2: 5'-TGAGGAAGCTGAGGAGGCGG-3',
gRNA3: 5'-GGCGGCGGCTGAGGAAGCTG-3',
gRNA4: 5'-GAAGGACTTGAGGGACTCGA-3',
gRNA5: 5'-TTCATTGCCCCGGTGCTGAG-3',
gRNA6: 5'-GGCTGAGGAAGCTGAGGAGG-3',
gRNA7: 5'-GACCCTGGAAAAGCTGATGA-3',
gRNA8: 5'-GGAGACCGCCATGGCGACCC-3',
gRNA9: 5'-CAGCTTTTCCAGGGTCGCCA-3',
gRNA10: 5'-CTTTTCCAGGGTCGCCATGG-3',
gRNA11: 5'-AGGCCTTCATCAGCTTTTCC-3',
gRNA12: 5'-GAGTCGGCCCGAGGCCTCCG-3',
gRNA13: 5'-GGCGGCTGAGGAAGCTGAGG-3',
gRNA14: 5'-AGCGGGCCCAAACTCACGGT-3',
gRNA15: 5'-AGCAGCGGCTGTGCCTGCGG-3',
gRNA16: 5'-GGAAGCTGAGGAGGCGGCGG-3',
gRNA17: 5'-GCCGGGACAGGGAGCTGCAG-3', and
gRNA18: 5'-TGAGGAGGCGGCGGCGGCGG-3'.

As an embodiment of the present disclosure, the *HTT* gene-targeted gRNA plasmid is a combination of at least one of gRNA1, gRNA4, gRNA5, and gRNA7-12 with at least one of gRNA2, gRNA3, gRNA6, and gRNA13-18.

### <Second Aspect>

The present disclosure relates to a preparation method of the lentivirus-like particle, including the following steps: transfecting plasmids carrying genomic sequences of a lentiviral vector into a virus-producing cell (collecting, concentrating, and purifying a resulting supernatant) to produce the lentivirus-like particle, where the genomic sequences of the lentiviral vector are located on (1) a plasmid expressing an envelope protein, (2) a plasmid expressing a lentiviral GagPol polyprotein including an RNA binding protein, (3) a plasmid expressing a lentiviral GagPol polyprotein, (4) a helper plasmid pRSV-Rev, and (5) a plasmid expressing the Cas9 protein required to produce the RNP complex and (6) a plasmid expressing HTT gRNA including an RNA stem-loop structure recognizable by the RNA binding protein, or (5) a plasmid expressing the Cas9 protein required to produce the RNP complex and the HTT gRNA including the RNA stem-loop structure recognizable by the RNA binding protein.

A backbone portion (a portion excluding the expressed core sequence) for the plasmids is not limited.

### <Third Aspect>

The present disclosure relates to a use of the lentivirus-like particle in preparation of a drug for treating HD.

### DESCRIPTION OF THE DRAWINGS

Other features, objectives, and advantages of the present disclosure will become more apparent by reading the detailed description of non-limiting embodiments with reference to the following accompanying drawings.
FIG. 1 is a map of a plasmid for expressing a single gRNA;
FIG. 2 shows gene-editing efficiencies of VLP-HDs;
FIG. 3 shows a gene-editing effect of dual-target VLP-HD;
FIG. 4 is a schematic diagram of the knockout of an HTT protein with dual-target VLP-HD;
FIG. 5 is a schematic diagram of a structure of VLP-HD;
FIG. 6 shows efficiencies of a neuron-specific envelope protein-containing lentivirus for cells from different sources;
FIG. 7 shows a gene-editing effect of neuron-specific VLP-HD; and
FIG. 8 shows data of the improvement of motor coordination and balance in HD mice by VLP-HD-NVR-G.

### SPECIFIC IMPLEMENTATIONS

The present disclosure is described in detail below with reference to embodiments. The following embodiments will help those skilled in the art further understand the present disclosure, but will not limit the present disclosure in any way. It should be noted that those of ordinary skill in the art can further make several modifications and improvements without departing from the idea of the present disclosure. These all fall within the protection scope of the present disclosure.

### Example 1: Construction of VLP-CRISPR RNPs (single-target and dual-target)

In this example, a method for constructing CRISPR RNP-containing VLP was as follows: a plasmid expressing an envelope protein (pMD.2G), a plasmid expressing a lentiviral GagPol polyprotein including an RNA binding protein (pMS2M-PH-GagPol-D64V with a sequence shown in SEQ ID NO. 1), a plasmid expressing a wild-type (WT) lentiviral GagPol polyprotein (pMDlg/PRRE-D64V with a sequence shown in SEQ ID NO. 2), a helper plasmid (pRSV-REV), a plasmid expressing a Cas9 protein (pCMV-2NLS-Cas9 with a sequence shown in SEQ ID NO. 3), and a plasmid expressing gRNA including MS2 stemloop (pU6-Osp.gRNAMS2in with a sequence shown in SEQ ID NO. 4) were co-transfected into 293T cells, and a resulting supernatant was collected, concentrated, and purified. A concentration of VLP p24 was measured with a lentiviral titer ELISA kit for quantification.

Dual-target VLP-CRISPR RNP was conducted. pU6-Osp.gRNAMS2in targeting another site was added on the basis of the plasmid production above to produce a plasmid expressing two gRNAs. Alternatively, expression cassettes for two gRNAs were incorporated in a same plasmid, that is, expression cassettes for two gRNA sequences (U6-Osp.gRNAMS2in) were inserted in cis or trans into a plasmid pU6-Osp.gRNAMS2in to replace the original single-copy expression cassette.

### Example 2: Design of gRNA sequences and prediction of gene-editing efficiencies

The HTT gene was input into the CRISPRon (v1.0) web tool to design gRNAs and predict gene-editing efficiencies. gRNAs were selected from results. gRNA sequences in a 5' direction of a CAG repeat region on exon 1 of the HTT gene were selected, such as gRNA1, gRNA4, and gRNA 7-11. These gRNAs could be used to construct single-target VLP-HD, and could also be combined with the selected gRNAs in a 3' direction of the CAG repeat region (such as gRNA2, gRNA3, gRNA6, and gRNA13-18) to produce dual-target VLP-HD. gRNA sequences were selected from a 5'-UTR region (such as gRNA5 and gRNA12), and combined with gRNA2, gRNA3, gRNA6, or gRNA13-18 to produce dual-target VLP-HD. A predicted gene-editing efficiency of each gRNA was shown in the table below:

| gRNA No. | gRNA sequence 5'-3' | PAM | Predicted gene-editing efficiency (%) | |
|---|---|---|---|---|
| gRNA1 | GGCCTTCATCAGCTTTTCCA | GGG | 52.401 | SEQ ID NO.11 |
| gRNA2 | TGAGGAAGCTGAGGAGGCGG | CGG | 68.825 | SEQ ID NO.12 |
| gRNA3 | GGCGGCGGCTGAGGAAGCTG | AGG | 68.463 | SEQ ID NO.13 |
| gRNA4 | GAAGGACTTGAGGGACTCGA | AGG | 60.817 | SEQ ID NO.14 |
| gRNA5 | TTCATTGCCCCGGTGCTGAG | CGG | 68.594 | SEQ ID NO.15 |
| gRNA6 | GGCTGAGGAAGCTGAGGAGG | CGG | 60.013 | SEQ ID NO.16 |
| gRNA7 | GACCCTGGAAAAGCTGATGA | AGG | 66.207 | SEQ ID NO.17 |
| gRNA8 | GGAGACCGCCATGGCGACCC | TGG | 62.471 | SEQ ID NO.18 |
| gRNA9 | CAGCTTTTCCAGGGTCGCCA | TGG | 49.442 | SEQ ID NO.19 |
| gRNA10 | CTTTTCCAGGGTCGCCATGG | CGG | 38.418 | SEQ ID NO.20 |
| gRNA11 | AGGCCTTCATCAGCTTTTCC | AGG | 13.299 | SEQ ID NO.21 |
| gRNA12 | GAGTCGGCCCGAGGCCTCCG | GGG | 60.424 | SEQ ID NO.22 |
| gRNA13 | GGCGGCTGAGGAAGCTGAGG | AGG | 64.402 | SEQ ID NO.23 |
| gRNA14 | AGCGGGCCCAAACTCACGGT | CGG | 63.217 | SEQ ID NO.24 |
| gRNA15 | AGCAGCGGCTGTGCCTGCGG | CGG | 62.264 | SEQ ID NO.25 |
| gRNA16 | GGAAGCTGAGGAGGCGGCGG | CGG | 59.637 | SEQ ID NO.26 |
| gRNA17 | GCCGGGACAGGGAGCTGCAG | CGG | 59.636 | SEQ ID NO.27 |
| gRNA18 | TGAGGAGGCGGCGGCGGCGG | CGG | 58.999 | SEQ ID NO.28 |

The different gRNA sites demonstrate different gene-editing efficiencies. The selected gRNA sequences all have a software-predicted efficiency of greater than 50% other than gRNA9-11.

### Example 3: Gene-editing efficiencies of single-target VLP-HDs for the HTT gene and verification of efficiencies of HTT gRNAs

1. Production of VLP-HDs: The VLP-HDs carried Cas9:gRNA RNP targeting the *HTT* gene. Plasmids pMD.2G, pMS2M-PH-GagPol-D64V, pMDlg/PRRE-D64V, pRSV-REV, pCMV-2NLS-Cas9, and pU6-Osp.gRNAMS2in-HTT were co-transfected into 293T cells, and a resulting supernatant was collected, concentrated, and purified to obtain VLP particles. A concentration of VLP p24 was measured with a lentiviral titer ELISA kit.

A plasmid pU6-Osp.gRNAMS2in-HTT was constructed. Several gRNA sequences targeting the HTT gene were selected, which were as follows: gRNA1: 5'-GGCCTTCATCAGCTTTTCCA-3' (PAM: GGG); gRNA2: 5'-TGAGGAAGCTGAGGAGGCGG-3' (PAM : CGG); gRNA3: 5'-GGCGGCGGCTGAGGAAGCTG-3' (PAM: AGG); gRNA4: 5'-GAAGGACTTGAGGGACTCGA-3' (PAM: AGG); gRNA5: 5'-TTCATTGCCCCGGTGCTGAG-3' (PAM : CGG); gRNA12:5'-GAGTCGGCCCGAGGCCTCCG-3' (PAM : GGG); and gRNA15:5'-AGCAGCGGCTGTGCCTGCGG -3' (PAM: CGG).

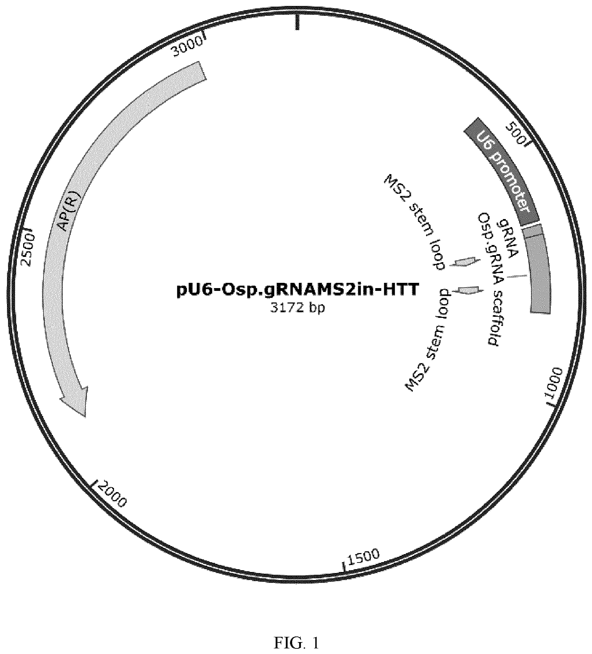

The above gRNA sequences were each added between a promoter and a gRNA scaffold of a plasmid pU6-Osp.gRNAMS2in to construct plasmids pU6-Osp.gRNAMS2in-HTT1, pU6-Osp.gRNAMS2in-HTT2, pU6-Osp.gRNAMS2in-HTT3, pU6-Osp.gRNAMS2in-HTT4, pU6-Osp.gRNAMS2in-HTT5, pU6-Osp.gRNAMS2in-HTT12, and pU6-Osp.gRNAMS2in-HTT15. A schematic diagram of a plasmid carrying gRNA was shown in FIG. 1 (the gRNA shown in this figure could be replaced with different gRNAs). The plasmid could be produced through gene synthesis based on a nucleotide sequence. VLPs produced using these plasmids were VLP-HD1, VLP-HD2, VLP-HD3, VLP-HD4, VLP-HD5, VLP-HD12, and VLP-HD15, respectively. VLP-HD1, VLP-HD4, and VLP-HD5 were single-target VLP-HDs with a function of polyQ knockout in the mutant HTT gene. VLP-HD2, VLP-HD3, and VLP-HD15 needed to be used in combination with any other VLP-HD to achieve the function of polyQ gene knockout.

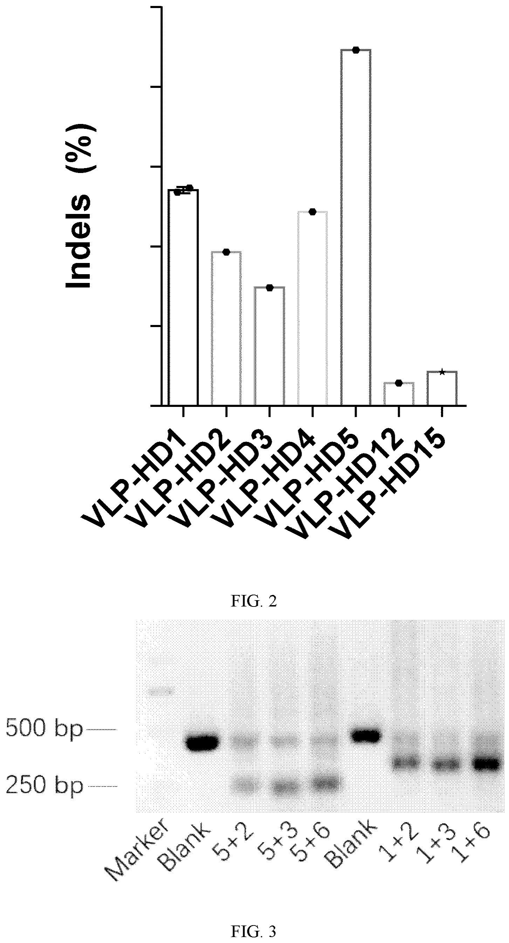

2. Verification of gene-editing efficiencies of VLP-HDs:
293T cells were inoculated into a 96-well plate at 2 × 10⁴ cells/well and cultured for 24 h. Then, the cells were infected with various VLP-HDs at 50 ng/well. At 72 h after the infection, cells were harvested and subjected to genome extraction. Primers (F: 5'-agggctgtcaatcatgctgg-3' and R: 5'-ggttgctgggtcactctgtc-3') were used to amplify a DNA fragment surrounding a target site through PCR. Sanger sequencing was conducted, and Indel was detected through TIDE (https://tide.nki.nl/). As shown in FIG. 2, the maximum gene-editing efficiency was close to 90%. In contrast to the predicted gene-editing efficiencies of gRNA sequences: A gene-editing efficiency of VLP-HD 1-4 was different from a predicted efficiency of gRNA1-4, but there was no significant difference. A gene-editing efficiency of VLP-HD5 (89.3%) was much higher than a predicted efficiency of gRNA15 (68.594%). Gene-editing efficiencies of VLP-HD12 and VLP-HD12 (Indel (insertion or deletion) efficiencies were 5.8% and 8.5%, respectively) were far lower than predicted efficiencies of gRNA 12 and gRNA 15 (which were 60.424% and 62.264%, respectively, as shown in Example 2).

### Example 4: Knockout of the HTT gene with dual-target VLP-HDs

1. Production of dual-target VLP-HDs: The VLP-HD carried CRISPR RNP targeting two HTT gene loci. Plasmids pMD.2G, pMS2M-PH-GagPol-D64V, pMDlg/PRRE-D64V, pRSV-REV, and pCMV-2NLS-Cas9 and two plasmids pU6-Osp.gRNAMS2in-HTT expressing HTT gRNA were co-transfected into 293T cells, and a resulting supernatant was collected, concentrated, and purified to obtain VLP particles. A concentration of VLP p24 was measured with a lentiviral titer ELISA kit.

Combinations of the two plasmids expressing HTT gRNA were gRNA1 + gRNA2, gRNA1 + gRNA3, gRNA1 + gRNA6, gRNA5 + gRNA2, gRNA5 + gRNA3, and gRNA5 + gRNA6. pU6-Osp.gRNAMS2in-HTT5 expressed gRNA5: 5'-TTCATTGCCCCGGTGCTGAG-3' (PAM: CGG). pU6-Osp.gRNAMS2in-HTT6 expressed gRNA6: 5'-GGCTGAGGAAGCTGAGGAGG-3' (PAM: CGG). These plasmids could be produced through gene synthesis based on nucleotide sequences.

2. Verification of gene cleavage by dual-target VLP-HDs:
HeLa cells were inoculated into a 96-well plate at 2 × 10⁴ cells/well and cultured for 24 h. Then, the cells were infected with VLP-HDs at 100 ng/well. At 72 h after the infection, cells were harvested and subjected to genome extraction. Primers (F: 5'-GGACGGGTCCAAGATGGACG-3' and R: 5'-caaactcacGGTCGGTGCAG-3') were used to amplify a DNA fragment surrounding a target site through PCR, and agarose gel electrophoresis was conducted. Due to the dual-target cleavage, a fragment including a CAG trinucleotide repeat would be removed, and it was expected to produce two DNA fragments, including the original fragment and a cleaved fragment. For gRNA1 + gRNA2, it was expected to produce 410 bp + 282 bp bands. For gRNA1 + gRNA3, it was expected to produce 410 bp + 273 bp bands. For gRNA1 + gRNA6, it was expected to produce 410 bp + 279 bp bands. For gRNA5 + gRNA2, it was expected to produce 410 bp + 208 bp bands. For gRNA5 + gRNA3, it was expected to produce 410 bp + 199 bp bands. For gRNA5 + gRNA6, it was expected to produce 410 bp + 205 bp bands. As shown in FIG. 3, after cells were infected with VLP-HD, a cleaved DNA band was consistent with an expected band, and a fragment including a CAG trinucleotide repeat was removed, indicating the gene-editing ability of VLP-HD. When VLP-HD was used for *mHTT* gene editing, all CAG trinucleotide fragments could be removed, thereby preventing the production of PolyQ to cause protein aggregation.

### Example 5: Knockout of the HTT protein with dual-target VLP-HDs

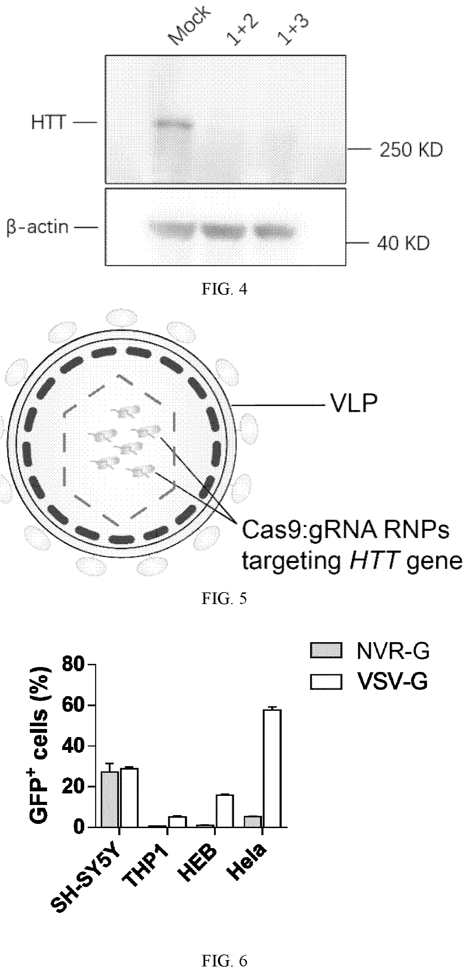

VLP-HD (gRNA1 + gRNA2) and VLP-HD (gRNA1 + gRNA3) were produced by the method in Example 3, and then used to infect HeLa cells. After 5 d of infection, a cell pellet was collected and lysed with radioimmunoprecipitation assay (RIPA) (including 1% of phenylmethylsulfonyl fluoride (PMSF)). A 10% loading buffer was added, and incubation was conducted in a metal bath at 98°C for 10 min to produce a sample. The sample was subjected to Western Blot analysis. Incubation was conducted overnight with anti-Huntingtin (3E10) and anti-β-actin antibodies as primary antibodies. Incubation was then conducted with anti-Mouse IgG as a secondary antibody at room temperature. Then a developing solution was added for development. As shown in FIG. 4, the HTT protein was knocked out after the *HTT* gene was cleaved with VLP-HD.

A schematic diagram of a structure of VLP-HD was shown in FIG. 5. As shown in FIG. 5, VLP-HD was a structure in which Cas9:gRNA RNP was packaged with a lentivirus-like particle (VLP) shell, where RNA was not limited to one type, and there was no RNA gene of the traditional lentiviral vector in the VLP.

### Example 6: Neuron-specific infectivity of the NVR-G envelope protein

1. A plasmid pNVR-G expressing a fusion protein NVR-G was constructed. A gene (with a sequence shown in SEQ ID NO. 29) expressing NVR-G was replaced with a gene expressing VSV-G in the pMD.2G plasmid to produce a plasmid pNVR-G with a sequence shown in SEQ ID NO. 5.
2. A method for producing a lentivirus expressing GFP and having an NVR-G protein-containing envelope was as follows: A plasmid expressing an envelope protein (pNVR-G), a plasmid expressing a WT lentiviral GagPol polyprotein (pMDlg/PRRE-D64V with a sequence shown in SEQ ID NO. 2), a helper plasmid (pRSV-REV), and a plasmid expressing a GFP protein (pCCL-PGK-eGFP with a sequence shown in SEQ ID NO. 6) were co-transfected into 293T cells, and a resulting supernatant was collected, concentrated, and purified. A concentration of VLP p24 was measured with a lentiviral titer ELISA kit for quantification.
3. A method for producing a lentivirus expressing GFP and having an VSV-G protein-containing envelope was as follows: A plasmid expressing an envelope protein (pMD.2G), a plasmid expressing a WT lentiviral GagPol polyprotein (pMDlg/PRRE-D64V), a helper plasmid (pRSV-REV), and a plasmid expressing a GFP protein (pCCL-PGK-eGFP) were co-transfected into 293T cells, and a resulting supernatant was collected, concentrated, and purified. A concentration of VLP p24 was measured with a lentiviral titer ELISA kit for quantification.
4. THP-1 (human monocytic leukemia cells), SH-SY5Y (human neuroblastoma cells), HEB (human astrocyte cells), and HeLa cell lines were each inoculated into a 48-well plate at 4 × 10⁴ cells/well. 24 h later, each cell line was infected with a lentivirus carrying the NVR-G envelope protein and a lentivirus carrying the VSV-G envelope protein at 20 ng. 48 h later, a proportion of cells expressing GFP in each group was detected by flow cytometry (BD & LSR Fortessa).

Experimental results were shown in FIG. 6. The lentivirus carrying the VSV-G envelope protein exhibited broad-spectrum infectivity, and could infect various types of cells generally with high infection efficiency. The lentivirus carrying the NVR-G envelope protein demonstrated high infection efficiency only when infecting the neuron line SH-SY5Y. VLP-HD required the editing of the *mHTT* gene in neurons to play a therapeutic role. Lentiviral particles including the NVR-G envelope protein could specifically infect neurons. Therefore, the lentivirus-like particle VLP-HD-NVR-G for treating HD that was produced with the NVR-G envelope protein could exhibit specified targetability in infecting neurons.

### Example 7: Editing of the HTT gene by VLP-HD-NVR-G

According to the method in Example 3, the plasmid pMD.2G was replaced with the plasmid pNVR-G to produce VLP-HD-NVR-G (gRNA1 + gRNA2). A gene-editing efficiency was verified using 293T cells.

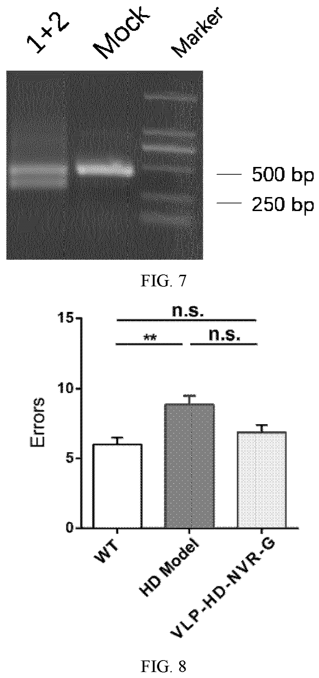

293T cells were inoculated into a 96-well plate at 2 × 10⁴ cells/well and cultured for 24 h. Then, the cells were infected with 100 ng of VLP-HD-NVR-G (gRNA1 + gRNA2). At 72 h after the infection, cells were harvested and subjected to genome extraction. Primers (F: 5'-GGACGGGTCCAAGATGGACG-3' and R: 5'-caaactcacGGTCGGTGCAG-3') were used to amplify a DNA fragment surrounding a target site through PCR, and agarose gel electrophoresis was conducted. Due to the dual-target cleavage, a fragment including a CAG trinucleotide repeat would be removed, and it was expected to produce two DNA fragments, including the original fragment and a cleaved fragment. For gRNA1 and gRNA2, it was expected to produce 410 bp + 282 bp bands. As shown in FIG. 7, PCR fragments for a gene-edited sample (lanes 1 + 2 in FIG. 7) indicated two expected bands.

### Example 8: Efficacy test for VLP-HD-NVR-G to treat HD mice

To investigate the potential of VLP-HD to alleviate the abnormal manifestations of HD, the *in vivo* efficacy and safety studies were carried out using an HD-specific mouse model. In this model, exon 1 of the endogenous mouse Htt was replaced with exon 1 of the human mutant Htt, and there were approximately 190 CAG repeats. One of the advantages of the mouse model is the high expression level of the mutant huntingtin protein, which makes the rapid onset and progression of HD symptoms possible. As a result, this mouse model becomes a useful model for studying the early stage of a disease and testing. HD mice undergo motor and balance disorders. The motor coordination and balance could be assessed through a beam balance test. Mice were allowed to walk 1 m on a gridded beam. During walking, paws of mice may fall off or slip between steel wires. If this circumstance happened, an error would be recorded. A total number of steps and a total time were recorded. 8-week-old HD mice were each intracranially injected with VLP-HD-NVR-G. Mice in a control group were each injected with phosphate-buffered saline (PBS). 4 weeks later, results were analyzed. Data showed that, in the beam balance test, the untreated HD mice (HD Model) exhibited significantly more foot-slip errors than WT mice, with a significant difference (**P < 0.01). After the treatment with VLP-HD-NVR-G, mice behaved with no significant difference from WT mice (n.s.) (FIG. 8). It can be seen that VLP-HD can improve the motor coordination and balance of HD mice.

Compared with the prior art, the present disclosure has the following beneficial effects:
1) The VLP-HD in the present disclosure is produced by encapsulating RNP including a Cas9 protein and a gRNA with a lentivirus-like particle. After infecting cells, VLP-HD can efficiently achieve the targeted knockout of the mutant HTT gene to prevent the production of the mutant HTT protein with PolyQ at source, thereby eliminating the etiology of HD at a genetic level and achieving the etiological therapy for HD.
2) The VLP-HD in the present disclosure can be either single-target VLP that delivers a single CRISPR/Cas9 system to knock out the gene through a frameshift mutation of the *mHTT* gene, or dual-target VLP that delivers two CRISPR/Cas9 systems to delete disease-causing CAG trinucleotide repeats in the *mHTT* gene, thereby completely avoiding the production of PolyQ.
3) Since the VLP-HD delivers an RNP structure produced from a Cas9 protein and a gRNA, CRISPR/Cas9 can be degraded by intracellular proteases and nucleases after completing the gene cleavage in cells. Therefore, the gene editing by VLP-HD is transient. The transient presence of CRISPR/Cas9 greatly reduces the off-target risk.

The specific embodiments of the present disclosure are described above. It should be understood that the present disclosure is not limited to the above specific implementations, and a person skilled in the art can make various variations or modifications within the scope of the claims without affecting the essence of the present disclosure.

## Claims

1. A lentivirus-like particle produced by encapsulating an RNP complex comprising a Cas9 protein and an *HTT* gene-targeted gRNA with a virus-like particle (VLP) vector.

2. The lentivirus-like particle according to claim 1, wherein gene sequences expressing components in the VLP vector are located on (1) a plasmid expressing an envelope protein, (2) a plasmid expressing a lentiviral GagPol polyprotein comprising an RNA binding protein, (3) a plasmid expressing a lentiviral GagPol polyprotein, (4) a helper plasmid pRSV-Rev, (5) a plasmid expressing the Cas9 protein required to produce the RNP complex, and (6) a plasmid expressing gRNA comprising an RNA stem-loop structure recognizable by the RNA binding protein, respectively.

3. The lentivirus-like particle according to claim 1, wherein the envelope protein among the components in the VLP vector comprises VSV-G, a CD4-recognizing protein, a CD8-recognizing protein, RD114, a baboon endogenous retrovirus envelope protein, or a modified envelope protein with cell-specific infectivity; the amino acid sequence of the VSV-G is shown in SEQ ID NO. 7; the modified envelope protein with cell-specific infectivity is an NVR-G protein with neuron-specific infectivity; and the amino acid sequence of the NVR-G protein is shown in SEQ ID NO. 8.

4. The lentivirus-like particle according to claim 1, wherein the lentiviral GagPol polyprotein comprising the RNA binding protein has the amino acid sequence shown in SEQ ID NO. 9; and the scaffold sequence of the gRNA comprising the RNA stem-loop structure recognizable by the RNA binding protein is shown in SEQ ID NO. 10.

5. The lentivirus-like particle according to claim 1, wherein a targeting site of a guide sequence carried by the *HTT* gene-targeted gRNA is any site on the *HTT* gene.

6. The lentivirus-like particle according to claim 1, wherein the *HTT* gene-targeted gRNA is gRNA1: 5'-GGCCTTCATCAGCTTTTCCA-3',
gRNA4: 5'-GAAGGACTTGAGGGACTCGA-3',
gRNA7: 5'- GACCCTGGAAAAGCTGATGA-3',
gRNA8: 5'- GGAGACCGCCATGGCGACCC-3',
gRNA9: 5'- CAGCTTTTCCAGGGTCGCCA-3',
gRNA10: 5'-CTTTTCCAGGGTCGCCATGG-3' or
gRNA11: 5'- AGGCCTTCATCAGCTTTTCC-3'.

7. The lentivirus-like particle according to claim 1, wherein the *HTT* gene-targeted gRNA comprises at least two selected from the following sequences:
gRNA1: 5'-GGCCTTCATCAGCTTTTCCA-3',
gRNA2: 5'-TGAGGAAGCTGAGGAGGCGG-3',
gRNA3: 5'-GGCGGCGGCTGAGGAAGCTG-3',
gRNA4: 5'-GAAGGACTTGAGGGACTCGA-3',
gRNA5: 5'-TTCATTGCCCCGGTGCTGAG-3',
gRNA6: 5'-GGCTGAGGAAGCTGAGGAGG-3',
gRNA7: 5'-GACCCTGGAAAAGCTGATGA-3',
gRNA8: 5'-GGAGACCGCCATGGCGACCC-3',
gRNA9: 5'-CAGCTTTTCCAGGGTCGCCA-3',
gRNA10: 5'-CTTTTCCAGGGTCGCCATGG-3',
gRNA11: 5'-AGGCCTTCATCAGCTTTTCC-3',
gRNA12: 5'-GAGTCGGCCCGAGGCCTCCG-3',
gRNA13: 5'-GGCGGCTGAGGAAGCTGAGG-3',
gRNA14: 5'-AGCGGGCCCAAACTCACGGT-3',
gRNA15: 5'-AGCAGCGGCTGTGCCTGCGG-3',
gRNA16: 5'-GGAAGCTGAGGAGGCGGCGG-3',
gRNA17: 5'-GCCGGGACAGGGAGCTGCAG-3', and
gRNA18: 5'-TGAGGAGGCGGCGGCGGCGG-3'.

8. The lentivirus-like particle according to claim 7, wherein the *HTT* gene-targeted gRNA plasmid is a combination of at least one of gRNAI, gRNA4, gRNA5, and gRNA7-12 with at least one of gRNA2, gRNA3, gRNA6, and gRNA13-18.

9. A preparation method of the lentivirus-like particle according to claim 1, comprising the following steps: co-transfecting a plasmid expressing an envelope protein, a plasmid expressing a lentiviral GagPol polyprotein comprising an RNA binding protein, a plasmid expressing a lentiviral GagPol polyprotein, a helper plasmid pRSV-Rev, and a plasmid expressing the Cas9 protein required to produce the RNP complex and a plasmid expressing HTT gRNA comprising an RNA stem-loop structure recognizable by the RNA binding protein, or a plasmid expressing the Cas9 protein required to produce the RNP complex and the HTT gRNA comprising the RNA stem-loop structure recognizable by the RNA binding protein into a virus-producing cell; and collecting, concentrating, and purifying a resulting supernatant to produce the lentivirus-like particle.

10. A use of the lentivirus-like particle according to claim 1 in preparation of a drug for treating Huntington's disease (HD).
